# EUROPEAN PATENT APPLICATION

(11) **EP 0 638 314 A1**
(43) Date of publication of application: **15.02.1995**
(21) Application number: 94901020.1
(22) Date of filing: 30.11.1993
(51) Int. Cl.: A61K 37/47, A61L 2/18, A61L 2/04

(54) **PROCESS FOR PRODUCING PLASMINOGEN-CONTAINING COMPOSITION**

(30) Priority: 01.12.1992 JP 343637/92
(71) Applicant: THE GREEN CROSS CORPORATION, Osaka-shi Osaka 541 (JP)
(72) Inventor: MOTIZUKI, Shinobu, The Green Cross Corporation, Hirakata-shi, Osaka 573 (JP); KOBAYASHI, Takashi, The Green Cross Corporation, Hirakata-shi, Osaka 573a 573 (JP); TANAKA, Kenji, The Green Cross Corporation, Hirakata-shi, Osaka 573 (JP)
(74) Representative: Hansen, Bernd, Dr. Dipl.-Chem.
(86) International application number: JP9301744
(87) International publication number: WO9412208

(57) **Abstract**

A process for producing a pharmaceutically safer plasminogen-containing composition by efficiently deactivating contaminant viruses while minimizing the loss of plasminogenic activity. The process comprises treating a plasminogen-containing composition which is likely to be contaminated by viruses with a trialkyl phosphate in solution and eliminating the phosphate. Preferably the phosphate treatment is conducted under a weakly acidic condition. The elimination of the phosphate is followed by freeze drying and heat treatment in a dried state.

## Description

### TECHNICAL FIELD

This invention relates to a process for producing a high activity plasminogen-containing composition which comprises substantially inactivating viruses in a plasminogen-containing composition with a possibility of having viral contamination.

### BACKGROUND ART

When plasminogen is activated by a plasminogen activator such as urokinase or the like, it is converted into plasmin which lyses fibrin to cause fibrinolysis. Because of this, a possibility has been noticed for the clinical application of plasminogen as a drug for the treatment of fibrinolytic systems (a thrombolytic agent). Especially, great attention has been focused on the usefulness of a lysyl type plasminogen whose N-terminal is a lysyl residue.

The lysyl type plasminogen is formed from a glutamyl type plasminogen, namely a plasminogen whose N-terminal is a glutamyl residue, when it is cut at a site between the 76 and 77 position lysyl residues counting from the N-terminal.

Since plasminogen is derived from human plasma, every plasminogen-containing composition has a possible danger of contamination with viruses such as hepatitis and AIDS viruses. Accordingly, with the aim of preventing transmission of such viruses, several processes have been proposed in which a plasma protein-containing composition is heated under a liquid condition (JP-A-55-145615, JP-A-56-139422 and JP-A-56-106594, etc.), heated under a dry condition (International Patent Application published in Japan No. 58-500548, etc.) or contact-treated with trialkyl phosphate (JP-A-60-51116, etc.). However, it is probable that thermally stable viruses remain after the heat treatment and viruses having no envelope remain after the trialkyl phosphate treatment. In addition, the trialkyl phosphate treatment has another problem in that the protein activity decreases during the treatment.

In order to overcome these problems, some processes have been proposed such as a process in which a heat treatment is carried out under a dry condition after a contact-treatment with trialkyl phosphate (EP-A-378208) and a process in which a contact-treatment with trialkyl phosphate and a heat treatment are simultaneously carried out under a liquid condition (JP-A-2-180833).

Taking the abovementioned problems into consideration, the present inventors have conducted studies on the development of a process for producing plasminogen-containing compositions. Accordingly, the present invention provides a process for producing a plasminogen-containing composition having improved safety as a pharmaceutical preparation, in which the loss of plasminogen activity is minimized and contaminated viruses are inactivated efficiently.

### DISCLOSURE OF THE INVENTION

The present invention relates to a process for producing a plasminogen-containing composition in which viruses are inactivated, which comprises (1) contact-treating a plasminogen-containing composition having a possibility of viral contamination with trialkyl phosphate under a liquid condition, and
(2) removing said trialkyl phosphate.

The plasminogen to be applied to the process of the present invention is not particularly limited and can be obtained from plasma, tissue, or by recombinant DNA technology or tissue culturing.

The plasminogen-containing composition (liquid state) to be used as a starting material for the process of the present invention is not particularly limited. Examples thereof include serum, plasma, ascites, placenta extract, placental tissue extract, the fraction II + III or III obtained from plasma by Cohn's low temperature alcohol fractionation method, a solution comprising a fraction obtained from plasma or a tissue extract obtained by various fractionation methods, a cultured broth obtained by recombinant host culture or tissue culture, and a commercially available plasminogen preparation (in a liquid state).

In addition, the purification degree of the plasminogen-containing composition at the time of its contact with the trialkyl phosphate of the present invention is not particularly limited but such a composition purified to a certain level can be used. Then, the contact with trialkyl phosphate may be carried out in either the isolation step or the purification step for the plasminogen.

Though not particularly limited, suitable examples of trialkyl phosphate to be used in the present invention include tri-(n-butyl) phosphate, tri-(tert-butyl) phosphate, tri-(n-hexyl) phosphate, tri-(2-ethylhexyl) phosphate, tri-(n-decyl) phosphate and the like. Particularly preferred trialkyl phosphate is tri-(n-butyl) phosphate (to be referred to as "TNBP" hereinafter). A mixture of two or more trialkyl phosphates may also be used.

Trialkyl phosphate of the present invention may be used in an amount of from 0.01 to 10% (w/v), preferably from about 0.1 to 3% (w/v).

Trialkyl phosphate may be used alone or together with a surface active agent. Preferably, trialkyl phosphate may be used in combination with a surface active agent. The surface active agent may be added to the plasminogen-containing composition at an optional step before, during or after the composition is contacted with trialkyl phosphate. The function of the surface active agent is to promote contact of viruses in the plasminogen-containing composition with the trialkyl phosphate.

Illustrative examples of the surface active agent include polyoxyethylene derivatives of fatty acids and partial esters of sorbitol anhydrides such as Tween 80, Tween 20 and polysorbate 80 and nonionic oil soluble rinsing agents such as Triton X100 (oxyethylated alkylphenol). Also useful are zwittergents which are synthetic zwitter-ion detergents known as sodium deoxycholate and sulfobetaine, such as N-dodecyl-N,N-dimethyl-2-ammonio-1-ethane sulfonate and homologs thereof, and nonionic detergents such as octyl-β,D-glucopyranoside and the like.

When the surface active agent is used, its amount is not critical but may be within the range of from about 0.001% to about 10% (w/v), preferably from about 0.01% to 3% (w/v).

The trialkyl phosphate treatment is especially useful for the inactivation of envelope-coated viruses such as hepatitis B virus, non-A non-B hepatitis virus, human immunodeficiency virus (HIV), vesicular stomatitis virus, sindbis virus and the like. Heat labile viruses may also be inactivated by heat treating for a sufficient time under a dry condition.

In the practice of the process of the present invention, the trialkyl phosphate treatment of the plasminogen-containing composition may be carried out at a temperature of from 0°C to 60°C, preferably from 20°C to 40°C, for a period of preferably 30 minutes or more, more preferably from 1 to 30 hours, most preferably from 3 to 10 hours. In addition, it is desirable to carry out this treatment under a slightly acidic condition, that is, at a pH value of from 4 to 6, preferably from 4.5 to 5.5.

Plasma and serum contain a glutamyl type plasminogen whose N-terminal is a glutamic acid residue, whereas the fraction II + III or III obtained by Cohn's low temperature alcohol fractionation method contains both glutamyl and lysyl type plasminogens. In order to obtain the lysyl type plasminogen, it is necessary to convert the glutamyl type plasminogen into the lysyl type. Such a conversion of glutamyl type into lysyl type can be effectively carried out by the trialkyl phosphate treatment under the aforementioned weakly acidic pH condition. In that case, the lysyl type plasminogen can be obtained without spoiling the plasminogen activity by carrying out the treatment in the absence of a protease inhibitor or in the presence of an appropriate concentration of the protease inhibitor.

On the other hand, the glutamyl type plasminogen can be obtained while its conversion into lysyl type is inhibited, by carrying out the treatment under a neutral or slightly alkaline pH condition. In that case, it is preferable to carry out the reaction in the presence of a protease inhibitor.

The protease inhibitor is not particularly limited provided that it is a substance which can substantially inhibit the protease activity. Examples thereof include basic amino acids such as -aminocaproic acid (EACA), lysine, arginine and the like, chemical substances such as diisopropylfluorophosphate, phenylmethane sulfonylfluoride and the like and proteins such as aprotinin and the like.

In general, trialkyl phosphate is removed after the trialkyl phosphate treatment. When a surface active agent and a stabilizer are used, they are also removed. The removal may be effected by optional means such as a method in which plasminogen is adsorbed by affinity or ion exchange chromatography, a method in which plasminogen is recovered by precipitation and a method in which plasminogen is separated from other components by gel filtration. These methods may be used in combination of two or more or by repeating the single method several times. Also, a heat treatment can be carried out at the time of the trialkyl phosphate removing treatment.

Preferably, in the trialkyl phosphate removing treatment, the plasminogen-containing composition may be treated finally by affinity chromatography, gel filtration or ion exchange chromatography. By carrying out affinity chromatography, gel filtration or ion exchange chromatography, trialkyl phosphate, surface active agent and stabilizer can be removed. In addition, even if strongly heat-resistant viruses such as non-envelope coat viruses remain, it is possible to remove all or most of these viruses by the affinity chromatography, gel filtration or ion exchange chromatography treatment. In the case of plasminogen, the affinity chromatography may be effected by the use of immobilized lysine.

When a heat treatment of a dried plasminogen-containing composition is carried out, the dry composition may be obtained by recovering plasminogen after the removal of trialkyl phosphate and the like, purifying it to a predetermined level of purity and then lyophilizing the product by known methods.

The heat treatment may be carried out at a temperature of generally from 30°C to 100°C, preferably from 55°C to 85°C, for generally from 3 to 200 hours, preferably from 10 to 100 hours. Also, it may be carried out in the presence of a stabilizer in order to protect the protein from heat. Examples of the stabilizer include human serum albumin, sugars, sugar alcohols, amino acids and the like.

### BEST MODE TO PRACTICE THE INVENTION

### Example 1

A paste extraction residue (100 g) of the fraction II + III obtained by Cohn's cold ethanol fractionation method was suspended in Tris-hydrochloride buffer (pH 8.3) containing 10 units/ml of aprotinin and 0.1 M of sodium chloride and, after a brief stirring, 5% ammonium sulfate was added to the resulting suspension, which was stirred and centrifuged to separate a supernatant. To the thus obtained supernatant was further added 30% ammonium sulfate, followed by stirring and then centrifugation to obtain 1 g of precipitate. The precipitate was suspended in 0.9% glycine solution (pH 7.2) containing 0.9% sodium chloride.

To the thus prepared plasminogen-containing solution were added TNBP (tri-(n-butyl) phosphate) to give a concentration of 0.3% and Tween 80 to give a concentration of 1%. One ml of the resulting solution was treated at pH 5 and at 30°C for 6 hours.

In accordance with the method of Deutsch, D.G. *et al*. [(*Science*, 170, 1095 (1970)], the plasminogen solution was applied to a lysine-Sepharose column (100 ml) which had been equilibrated in advance with 0.9% glycine solution (pH 7.2) containing 0.9% sodium chloride for adsorption of plasminogen. Then, the column was washed with 0.9% glycine solution (pH 7.2) containing 0.9% sodium chloride and 500 ml of 0.9% glycine solution (pH 7.2) containing 1 M sodium chloride. Thereafter, elution of adsorbed plasminogen was carried out with 0.9% glycine solution (pH 7.2) containing 0.25 M lysine.

By this treatment, TNBP and Tween 80 were removed. The thus obtained plasminogen was purified to a desired level of purity, dissolved in a solution to a predetermined concentration, dispensed into vials in small portions and then lyophilized. The thus lyophilized plasminogen preparation was subjected to a dry heat treatment at 60 to 80°C for 72 hours or more to produce a lysyl type plasminogen-containing composition in which viruses were removed or inactivated.

### Example 2

After the same TNBP/Tween 80 treatment as in Example 1, gel filtration was carried out using Sephacryl S-300 and 0.9% glycine solution (pH 7.2) containing 0.9% w/v of sodium chloride as a developing solution, followed by affinity chromatography using immobilized lysine in the same manner as in Example 1. Similar to the case of Example 1, a high activity lysyl type plasminogen-containing composition in which viruses were inactivated was prepared.

### Example 3

The pH conditions at the time of the same TNBP/Tween 80 treatment as in Example 1 and the conversion degree of glutamyl type plasminogen into lysyl type plasminogen were examined by repeating the procedure of Example 1. The results are shown in Table 1.

**Table 1**

| pH | Before | | After | |
|---|---|---|---|---|
| | TNBP/Tween 80 treatment | | TNBP/Tween 80 treatment | |
| | Glutamyl type | Lysyl type | Glutamyl type | Lysyl type |
| 4.5 | 65% | 35% | 0% | 100% |
| 5.0 | 65% | 35% | 0% | 100% |
| 5.5 | 65% | 35% | 0% | 100% |
| 6.0 | 65% | 35% | 58% | 42% |
| 7.2 | 65% | 35% | 65% | 35% |

### Example 4

A paste extraction residue (1.1 kg) of the fraction II + III obtained by Cohn's cold ethanol fractionation method was suspended in Tris-hydrochloride buffer (pH 8.3) containing 10 units/ml of aprotinin and 0.1 M of sodium chloride, stirred briefly and then subjected to centrifugation and filtration to separate supernatant.

The supernatant was applied to lysine-Sepharose (3 liters) to effect adsorption of plasminogen. The resulting resin was washed with 0.9% glycine solution (pH 7.2) containing 0.9% sodium chloride and subsequently 0.9% glycine solution (pH 7.2) containing 1 M sodium chloride and then the adsorbed plasminogen was eluted with 0.9% glycine solution (pH 7.2) containing 0.25 M lysine.

The eluate was concentrated to obtain 250 ml of a concentrated solution.

To 1 ml of the thus prepared plasminogen-containing concentrate were added TNBP and Tween 80 to respective concentrations of 0.3% and 1%. The resulting solution (250 ml) was treated at pH 5 and at 30°C for 6 hours.

The thus treated plasminogen solution was subjected to gel filtration by applying it to a Sephacryl S300 column (10 liters) which had been equilibrated in advance with 0.9% glycine solution (pH 7.2) containing 0.9% sodium chloride. Similar to the case of Example 1, a high activity lysyl type plasminogen-containing composition in which viruses were inactivated was prepared.

### Example 5

A paste extraction residue (1.5 kg) of the fraction II + III obtained by Cohn's cold ethanol fractionation method was suspended in Tris-hydrochloride buffer (pH 8.3) containing 100 units/ml of aprotinin and 0.1 M of sodium chloride. After stirring briefly, the resulting mixture was subjected to centrifugation and filtration to separate supernatant.

The supernatant was applied to lysine-Sepharose (3 liters) to effect adsorption of plasminogen. The resulting resin was washed with 0.9% glycine solution (pH 7.2) containing 0.9% sodium chloride and subsequently 0.9% glycine solution (pH 7.2) containing 1 M sodium chloride and then the thus adsorbed plasminogen was eluted with 0.9% glycine solution (pH 7.2) containing 0.25 M lysine.

One hundred units/ml (final concentration) of aprotinin was added to the eluate and then concentrated to obtain 220 ml of a concentrated solution (250 ml).

To 1 ml of the thus prepared plasminogen-containing concentrate were added 0.3% of TNBP, 1% of Tween 80 and 100 units/ml (final concentration) of aprotinin. The resulting solution was treated at pH 7.2 and at 30°C for 6 hours.

The thus treated plasminogen solution was subjected to gel filtration by applying it to a Sephacryl S300 column (10 liters) which had been equilibrated in advance with 0.9% glycine solution (pH 7.2) containing 0.9% sodium chloride. Thus, a high activity plasminogen-containing composition was prepared, in which viruses were inactivated and 65% portion of the plasminogen was glutamyl type.

### INDUSTRIAL APPLICABILITY

According to the production process of the present invention, a plasminogen-containing composition in which viruses are inactivated can be produced efficiently without spoiling the plasminogen activity.

The trialkyl phosphate treatment poorly inactivates viruses having no envelope. However, according to the present invention such viruses can be inactivated efficiently through a step of heat treatment of the plasminogen-containing composition under a dry condition.

In addition, lysyl type plasminogen which is expected to be useful as a thrombolytic agent can be obtained by carrying out the trialkyl phosphate treatment step under a slightly acidic condition to efficiently convert glutamyl type plasminogen into lysyl type.

On the other hand, glutamyl type plasminogen can be obtained by carrying out this step under a neutral or alkaline pH condition in the presence of a protease inhibitor.

In consequence, the production process of the present invention provides a markedly desirable method as an industrial process for producing plasminogen-containing compositions having improved safety as pharmaceutical preparations and is especially useful for producing plasminogen preparations in which viruses have been inactivated.

## Claims

1. A process for producing a plasminogen-containing composition in which viruses are inactivated, which comprises
(1) contact-treating a plasminogen-containing composition having a possibility of viral contamination with trialkyl phosphate under a liquid condition, and
(2) removing said trialkyl phosphate.

2. The process for producing a plasminogen-containing composition according to claim 1, wherein said contact-treatment with a trialkyl phosphate is carried out under a slightly acidic condition.

3. The process for producing a plasminogen-containing composition according to claim 1, wherein said process further comprises (3) lyophilizing, and (4) heat-treating the plasminogen-containing composition under a dry condition.

4. The process for producing a plasminogen-containing composition according to claim 3, wherein said heat treatment is carried out to such an extent that non-envelope viruses are inactivated.

5. The process for producing a plasminogen-containing composition according to claim 4, wherein said heat treatment is carried out at a temperature of from 30 to 100°C for 3 to 200 hours.

6. The process for producing a plasminogen-containing composition according to claim 2, wherein said slightly acidic condition means pH 4 to 6.

7. The process for producing a plasminogen-containing composition according to claim 1, wherein said trialkyl phosphate treatment is carried out in the presence of a surface active agent.

8. The process for producing a plasminogen-containing composition according to claim 7, wherein said surface active agent is a polyoxyethylene derivative of a fatty acid, a partial ester of a sorbitol anhydride, a nonionic oil soluble rinsing agent, sodium deoxycholate, a synthetic zwitter-ion detergent or a homolog thereof, or a nonionic detergent.

9. The process for producing a plasminogen-containing composition according to claim 1, wherein said trialkyl phosphate is tri-(n-butyl) phosphate, tri-(tert-butyl) phosphate, tri-(n-hexyl) phosphate, tri-(2-ethylhexyl) phosphate or tri-(n-decyl) phosphate.

10. The process for producing a plasminogen-containing composition according to claim 1, wherein said trialkyl phosphate treatment is carried out at 0 to 60°C for 30 minutes or more.

11. The process for producing a plasminogen-containing composition according to claim 1, wherein said plasminogen is glutamyl type, lysyl type or a mixture thereof.

12. The process for producing a plasminogen-containing composition according to claim 1, wherein a lysyl type plasminogen-containing composition in which viruses are inactivated is produced by
(1) contact-treating a glutamyl type plasminogen-containing composition having a possibility of viral contamination with trialkyl phosphate under liquid and slightly acidic conditions, and
(2) removing said trialkyl phosphate.
